# EUROPEAN PATENT APPLICATION

(11) **EP 4 574 139 A1**
(43) Date of publication of application: **25.06.2025**
(21) Application number: 24221339.5
(22) Date of filing: 19.12.2024
(51) Int. Cl.: A61K 9/20, A61K 31/47

(54) **A FILM COATED TABLET COMPRISING IVACAFTOR**

(30) Priority: 20.12.2023 TR 202317799
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); GULER, Tolga, Istanbul (TR)
(74) Representative: Genç Ilhan, Oznur

(57) **Abstract**

The present invention relates to a film coated tablet comprising a solid dispersion having ivacaftor or a pharmaceutically acceptable salt thereof and a disintegrant, wherein the disintegrant is crospovidone. The present invention also relates to a simple, rapid, cost effective, time-saving and industrially convenient process of preparing the film coated tablet.

## Description

### Field of Invention

The present invention relates to a film coated tablet comprising a solid dispersion having ivacaftor or a pharmaceutically acceptable salt thereof and a disintegrant, wherein the disintegrant is crospovidone. The present invention also relates to a simple, rapid, cost effective, time-saving and industrially convenient process of preparing the film coated tablet.

### Background of the Invention

ivacaftor, also known as N-(2,4-di-tert-butyl-5-hydroxyphenyl)-1,4-dihydro-4-oxoquinoline-3-carboxamide, having the following Formula (I):

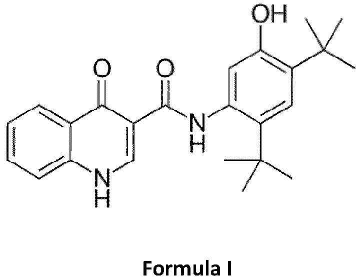

Ivacaftor is a white to off-white powder that is practically insoluble in water (<0.05 microgram/mL). Due to poor aqueous solubility, extensive formulation efforts were required and resulted in a spray-dried dispersion of Ivacaftor suitable for oral administration. Ivacaftor was approved by FDA and marketed by vertex pharma for the treatment of cystic fibrosis under the brand name KALYDECO^{®} in the form of 150 mg oral tablets. Kalydeco^{®} is indicated for the treatment of cystic fibrosis in patients age 6 years and older who have a G55ID mutation in the CFTR (cystic fibrosis transmembrane conductance regulator) gene.

Ivacaftor is a potentiator of the CFTR protein. The CFTR protein is a chloride channel present at the surface of epithelial cells in multiple organs. Ivacaftor facilitates increased chloride transport by potentiating the channel-open probability (or gating) of the CFTR protein.

The patent WO2022013360A1 disclose an immediate release tablet composition comprising free particles of ivacaftor a pH dependent polymer and surfactant extragranularly and intragranularly.

The patent US2015010628A1 disclose a solid dispersion of N-[2,4-Bis(1,1-dimethylethyl)-5-hydroxyphenyl]-1,4-dihydro-4-oxoquinoline-3-carboxamide including formulations of the solid dispersions into powders, granules and mini-tablets, methods for manufacturing and processing the powders and mini-tablets and methods for treating cystic fibrosis employing the pharmaceutical composition.

It is known in the prior art that ivacaftor has poor solubility, and there are patent applications written regarding this. However, a new formulation is still needed to address problems such as content uniformity and flowabilty that arise due to the high use of ivacaftor by weight in the formulation.

In this invention, we found that the use of ivacaftor with crospovidone overcomes the above-mentioned problems and provides additional advantages over the prior art.

### Detailed description of the Invention

The main object of the present invention is a film coated tablet formulation comprising ivacaftor which has the desired dissolution profile and content uniformity, flowability and compresibility.

Another object of the present invention is to provide a film coated tablet comprising ivacaftor which has high stability.

Another object of the present invention is to provide a preparation process of the film coated tablet formulation comprising ivacaftor which is simple and cost-effective process.

The rate of ivacaftor by weight is high in the formulation, we saw that it provides the desired dissolution profile with crospovidone while also ensuring that the active ingredient is homogeneous in the formulation. While this ensures content uniformity, its flowability is also improved. As a result, the formulation of ivacaftor with crospovidone both improved dissolution and helped maintain stability.

According to one embodiment of the invention, a film coated tablet comprising a solid dispersion of ivacaftor or a pharmaceutically acceptable salt thereof and a disintegrant, wherein the disintegrant is crospovidone.

According to one embodiment of the invention, the solid dispersion comprises ivacaftor, hypromellose acetate succinate and sodium lauryl sulfate.

According to one embodiment of the invention, the solid dispersion comprises between 70.0 % and 80.0 % ivacaftor by weight in the dispersion, between 20.0% and 25.0 % of hypromellose acetate succinate by weight in the dispersion, and 0.1 % and 1.0 % of sodium lauryl sulfate by weight in the dispersion.

According to one embodiment of the invention, the amount of a solid dispersion is between 25.0% and 45.0% by weight in the total formulation.

According to this embodiment of the present invention, the amount of crospovidone is between 1.0% and 10.0% by weight of the total formulation. Preferably, the amount of crospovidone is between 2.0% and 4.0% by weight of the total formulation. Also, used disintegrant in the specified range helps to provide the desired dissolution profile. Tablet brittleness increases when crospovidone is used above this ratio, so we have seen that these ratios also provide physical stability.

According to an embodiment of the present invention, the film coating tablet further comprises at least one pharmaceutically acceptable excipient which is selected from the group comprising diluents, binders, surfactants, glidants, lubricants or a mixture thereof.

Suitable diluents are selected from the group comprising lactose monohydrate, lactose, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or a mixture thereof.

According to this embodiment of the present invention, the amount of diluent is between 15.0% and 35.0% by weight of the total formulation. Preferably, the amount of diluent is between 20.0% and 30.0% by weight of the total formulation.

According to an embodiment of the present invention, the diluent is lactose monohydrate.

Suitable binders are selected from the group comprising microcrystalline cellulose, corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or a mixture thereof.

According to this embodiment of the present invention, the binder is microcrystalline cellulose.

According to this embodiment of the present invention, the amount of binder is between 20.0% and 40.0% by weight of the total formulation. Preferably, the amount of binder is between 25.0% and 35.0% by weight of the total formulation.

Suitable surfactants are selected from the group comprising sodium lauryl sulfate, cetostearyl alcohol, cetomacrogol emulsifying wax, gelatin, casein, docusate sodium, benzalkonium chloride, calcium stearate, polyethylene glycols, phosphates, polyoxyethylene sorbitan fatty acid esters, tragacanth, polyoxyethylene 20 stearyl ethers, polyoxyethylene alkyl ethers, polyoxyethylene castor oil derivatives, pegylated hydrogenated castor oils, sorbitan esters of fatty acids, Vitamin E or tocopherol derivatives, vitamin E TPGS, tocopheryl esters, lecithin, phospholipids and their derivatives, poloxamers, stearic acid, oleic acid, oleic alcohol, cetyl alcohol, mono and diglycerides, propylene glycol esters of fatty acids, ethylene glycol palmitostearate, polyoxylglycerides, propylene glycol monocaprylate, propylene glycol monolaurate, alkyl aryl polyether alcohols and polyglyceryl oleate or a mixture thereof.

According to an embodiment of the present invention, the surfactant is sodium lauryl sulfate.

According to this embodiment of the present invention, the amount of surfactant is between 0.01% and 5.0% by weight of the total formulation. Preferably, the amount of surfactant is between 0.1% and 2.0% by weight of the total formulation.

Suitable lubricants are selected from the group comprising sodium stearyl fumarate, fatty acid, stearic acid, magnesium stearate, calcium stearate, sodium stearate, stearic acid, glyceryl monostearate, hydrogenated oils (i.e. hydrogenated vegetable oil), polyethylene glycol, fatty alcohol, fatty acid ester, glyceryl behenate, mineral oil, vegetable oil, leucine, sodium benzoate, or a mixture thereof.

According to an embodiment of the present invention, the lubricant is sodium stearyl fumarate. It improves the powder processing properties of film coated tablet formulation. Also, it enhances powder flow by reducing the inter-particle friction. This ensures also good compressibility.

According to this embodiment of the present invention, the amount of lubricant is between 0.01% and 5.0% by weight of the total formulation. Preferably, the amount of lubricant is between 0.1% and 3.0% by weight of the total formulation.

Suitable glidants are selected from the group comprising colloidal anhydrous silica, magnesium oxide, magnesium silicate, hydrophobic colloidal silica, calcium phosphate, powdered cellulose or a mixture thereof.

According to an embodiment of the present invention, the glidant is colloidal anhydrous silica.

According to this embodiment of the present invention, the amount of glidant is between 0.01% and 5.0% by weight of the total formulation.

In the present invention, the tablet comprises film coating to protect the composition against the moisture and light to maintain the stability. Suitable film coating agents are selected from the group comprising polyvinyl alcohol (PVA), titanium dioxide, macrogol (PEG 3350), Indigo carmine aluminium lake, talc, carnauba wax, hydroxypropylmethylcellulose, polyethylene glycol (PEG), polyvinyl alcohol-polyethylene glycol copolymers, lecithin, polyvinylprolidone-vinyl acetate copolymer (PVP-VA), pigments, dyes, iron oxide or a mixture thereof.

According to this embodiment of the present invention, the amount of film coating agent is between 0.5% and 5.0% by weight of the total formulation.

According to this embodiment of the present invention, the film coated tablet comprises;
- The solid dispersion comprising ivacaftor
- Crospovidone
- Sodium stearyl fumarate

According to one embodiment of the present invention, the film coated tablet comprising ivacaftor or a pharmaceutically acceptable salt thereof is obtained by dry granulation process. The desired dissolution profile and stability of the film coated tablet is obtained and it has a simple preparation process suitable for industrial production.

*A process for preparing a film coated tablet comprising ivacaftor comprises the following steps;*
- Weighing the solid dispersion comprising ivacaftor and all excipients,
- Mixing lactose monohydrate, microcrystalline cellulose, crospovidone, sodium lauryl sulfate, colloidal silicon dioxide and solid dispersion comprising ivacaftor,
- Adding sodium stearyl fumarate is to the mixture and mixing,
- Compressing the resulting homogeneous mixture in accordance with the specifications specified for each tablet,
- Coating the tablets with film coating.

### Example 1; A film coated tablet

| **Ingredients** | **% by weight of the total tablet** | **% by weight of the total tablet** |
|---|---|---|
| Solid dispersion comprising ivacaftor | 34.8 | 25-45 |
| Lactose monohydrate | 27.7 | 15-35 |
| Microcrystalline cellulose | 29.6 | 20-40 |
| Crospovidone | 2.9 | 1-10 |
| Sodium lauryl sulfate | 0.5 | 0.01-5 |
| Colloidal Anhydrous Silica | 0.5 | 0.01-5 |
| Sodium stearyl fumarate | 1 | 0.01-5 |
| **Tablet core weight** | **97** | **90-99** |
| Film coating | 3 | 0.5-5 |
| - Polyvinyl alcohol | | |
| -Titanium dioxide | | |
| -Macrogol (PEG 3350) | | |
| -Indigo carmine aluminium lake | | |
| -Talk | | |
| -Carnauba wax | | |
| **Total Tablet** | **100** | **100** |

***A process for example 1;***
- Weighing the solid dispersion comprising ivacaftor and all excipients,
- Mixing lactose monohydrate, microcrystalline cellulose, crospovidone, sodium lauryl sulfate, colloidal silicon dioxide and solid dispersion comprising ivacaftor,
- Adding sodium stearyl fumarate is to the mixture and mixing,
- Compressing the resulting homogeneous mixture in accordance with the specifications specified for each tablet,
- Coating the tablets with film coating.

### Example 2; A solid dispersion

| Solid dispersion | **% by weight** |
|---|---|
| Ivacaftor | 76 |
| Hypromellose acetate succinate | 23.5 |
| Sodium lauryl sulfate | 0.5 |
| **Total** | **100** |

## Claims

1. A film coated tablet comprising a solid dispersion of ivacaftor or a pharmaceutically acceptable salt thereof and a disintegrant, wherein the disintegrant is crospovidone.

2. The film coated tablet according to claim 1, wherein amount of crospovidone is between 1.0% and 10.0% by weight of the total formulation.

3. The film coated tablet according to claim 1, wherein further comprises at least one pharmaceutically acceptable excipient which is selected from the group comprising diluents, binders, surfactants, glidants, lubricants or mixtures thereof.

4. The film coated tablet according to claim 3, wherein lubricants are selected from the group comprising sodium stearyl fumarate, fatty acid, stearic acid, magnesium stearate, calcium stearate, sodium stearate, stearic acid, glyceryl monostearate, hydrogenated oils (i.e. hydrogenated vegetable oil), polyethylene glycol, fatty alcohol, fatty acid ester, glyceryl behenate, mineral oil, vegetable oil, leucine, sodium benzoate, or a mixture thereof.

5. The film coated tablet according to claim 4, wherein lubricant is sodium stearyl fumarate.

6. The film coated tablet according to claim 4 or 5, wherein amount of lubricant is between 0.01% and 5.0% by weight of the total formulation.

7. The film coated tablet according to claim 3, wherein diluents are selected from the group comprising lactose monohydrate, lactose, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or a mixture thereof.

8. The film coated tablet according to claim 3 or 7, wherein diluent is lactose monohydrate.

9. The film coated tablet according to claim 3, wherein binders are selected from the group comprising microcrystalline cellulose, corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

10. The film coated tablet according to claim 3 or 9, wherein binder is microcrystalline cellulose.

11. The film coated tablet according to claim 3, wherein surfactants are selected from the group comprising sodium lauryl sulfate, cetostearyl alcohol, cetomacrogol emulsifying wax, gelatin, casein, docusate sodium, benzalkonium chloride, calcium stearate, polyethylene glycols, phosphates, polyoxyethylene sorbitan fatty acid esters, tragacanth, polyoxyethylene 20 stearyl ethers, polyoxyethylene alkyl ethers, polyoxyethylene castor oil derivatives, pegylated hydrogenated castor oils, sorbitan esters of fatty acids, Vitamin E or tocopherol derivatives, vitamin E TPGS, tocopheryl esters, lecithin, phospholipids and their derivatives, poloxamers, stearic acid, oleic acid, oleic alcohol, cetyl alcohol, mono and diglycerides, propylene glycol esters of fatty acids, ethylene glycol palmitostearate, polyoxylglycerides, propylene glycol monocaprylate, propylene glycol monolaurate, alkyl aryl polyether alcohols and polyglyceryl oleate or a mixture thereof.

12. The film coated tablet according to claim 3 or 11, wherein surfactant is sodium lauryl sulfate.

13. The film coated tablet according to claim 3, wherein glidants are selected from the group comprising colloidal anhydrous silica, magnesium oxide, magnesium silicate, hydrophobic colloidal silica, calcium phosphate, powdered cellulose or a mixture thereof.

14. The film coated tablet according to claim 3 or 13, wherein glidant is colloidal anhydrous silica.
